# EUROPEAN PATENT APPLICATION

(11) **EP 4 699 564 A1**
(43) Date of publication of application: **25.02.2026**
(21) Application number: 25192676.2
(22) Date of filing: 30.07.2025
(51) Int. Cl.: A61B 34/30, A61B 34/37, A61B 90/00, B25J 9/00, A61B 34/00

(54) **ROBOTIC SURGICAL SYSTEM, CONTROL METHOD FOR ROBOTIC SURGICAL SYSTEM, PROGRAM, AND STORAGE MEDIUM**

(30) Priority: 23.08.2024 JP 2024143201
(71) Applicant: KAWASAKI JUKOGYO KABUSHIKI KAISHA, Kobe-shi, Hyogo 650-8670 (JP)
(72) Inventor: TANI, Hidenori, Kobe-shi, Hyogo, 650-8670 (JP); KODAMA, Kazuki, Kobe-shi, Hyogo, 650-8670 (JP); YAMAMOTO, Daisuke, Kobe-shi, Hyogo, 650-8670 (JP)
(74) Representative: Hoffmann Eitle

(57) **Abstract**

A robotic surgical system (500) includes a surgical robot (100) including a robot arm (50) to allow a surgical instrument (1) to be attached thereto, an operation apparatus (200) including an operation unit (110) to receive an operation from an operator, the operation unit (110) including a drive (SM7a, SM7b, SM7c) to assist the operator in performing the operation, the operation apparatus (200) being configured to operate the surgical robot (100), and a control device (340) configured or programmed to set a plane (SU) when the operation unit (110) is moved such that the robot arm (50) is attempting to move out of a normal region, and control the drive (SM7a, SM7b, SM7c) to apply a force (PW) to the operation unit (110) when the operation unit (110) crosses the plane (SU).

## Description

### BACKGROUND OF THE INVENTION

### Field of the Invention

The present disclosure relates to a robotic surgical system, a control method for a robotic surgical system, a program, and a storage medium.

### Description of the Background Art

Conventionally, a robotic surgical system is known. The robotic surgical system disclosed in Japanese Patent No. 6309514 includes a robot arm including a surgical instrument, a handheld surgical user interface for an operator to operate the robot arm, and a feedback device that provides the operator with feedback related to the function of the surgical instrument. The feedback of the feedback device includes auditory feedback that provides the operator with feedback by sound, optical feedback that provides the operator with feedback by light, and tactile feedback that provides the operator with feedback by force.

In a robotic surgical system as described in Japanese Patent No. 6309514, when a robot arm is operated by a handheld surgical user interface, the robot arm may move out of a normal region. In such a case, it is necessary to return the robot arm to the normal region. In Japanese Patent No. 6309514, although the operator is provided with feedback by sound, light, force, etc., a warning is merely presented to the operator, and Patent No. 6309514 does not disclose anything about returning the robot arm to a normal region. It is desired to enable the robot arm to easily return to the normal region.

### SUMMARY OF THE INVENTION

The present disclosure aims to provide a robotic surgical system, a control method for a robotic surgical system, a program, and a storage medium that each enable a robot arm to easily return to a normal region.

A robotic surgical system according to a first aspect of the present disclosure includes a surgical robot including a robot arm to allow a surgical instrument to be attached thereto, an operation apparatus including an operation unit to receive an operation from an operator, the operation unit including a drive to assist the operator in performing the operation, the operation apparatus being configured to operate the surgical robot, and a control device configured or programmed to set a plane when the operation unit is moved such that the robot arm is attempting to move out of a normal region, and control the drive to apply a force to the operation unit when the operation unit crosses the plane.

The robotic surgical system according to the first aspect of the present disclosure includes the control device configured or programmed to set the plane when the operation unit is moved such that the robot arm is attempting to move out of the normal region, and control the drive to apply the force to the operation unit when the operation unit crosses the plane. Accordingly, the force acting on the operation unit allows the operator to know that the robot arm is attempting to move out of the normal region, and the force acting on the operation unit allows the operator to intuitively know the operation direction of the operation unit to return the robot arm to the normal region. Consequently, the robot arm can be easily returned to the normal region.

A control method for a robotic surgical system according to a second aspect of the present disclosure is a control method for a robotic surgical system including a surgical robot including a robot arm to allow a surgical instrument to be attached thereto, and an operation apparatus including an operation unit to receive an operation from an operator, the operation unit including a drive to assist the operator in performing the operation, the operation apparatus being configured to operate the surgical robot, and the control method includes receiving an operation on the operation unit, and setting a plane when the operation unit is moved such that the robot arm is attempting to move out of a normal region and controlling the drive to apply a force to the operation unit when the operation unit crosses the plane.

As described above, the control method for the robotic surgical system according to the second aspect of the present disclosure includes setting the plane when the operation unit is moved such that the robot arm is attempting to move out of the normal region and controlling the drive to apply the force to the operation unit when the operation unit crosses the plane. Accordingly, the force acting on the operation unit allows the operator to know that the robot arm is attempting to move out of the normal region, and the force acting on the operation unit allows the operator to intuitively know the operation direction of the operation unit to return the robot arm to the normal region. Consequently, it is possible to provide the control method for the robotic surgical system that enables the robot arm to easily return to the normal region.

A program according to a third aspect of the present disclosure is a program for the control method for the robotic surgical system according to the second aspect. Accordingly, similarly to the control method for the robotic surgical system according to the second aspect, it is possible to provide the program that enables the robot arm to easily return to the normal region.

A storage medium according to a fourth aspect of the present disclosure is configured to store the program according to the third aspect. Accordingly, similarly to the program according to the third aspect, it is possible to provide the storage medium that enables the robot arm to easily return to the normal region.

According to the present disclosure, it is possible to easily return the robot arm to the normal region.

The foregoing and other objects, features, aspects and advantages of the present disclosure will become more apparent from the following detailed description of the present disclosure when taken in conjunction with the accompanying drawings.

### BRIEF DESCRIPTION OF THE DRAWINGS

FIG. 1 is a diagram showing the configuration of a robotic surgical system according to an embodiment.
FIG. 2 is a diagram showing a display of a medical cart according to the embodiment.
FIG. 3 is a diagram showing the configuration of the medical cart according to the embodiment.
FIG. 4 is a diagram showing the configuration of a robot arm according to the embodiment.
FIG. 5 is a diagram showing an instrument.
FIG. 6 is a perspective view showing the configuration of an arm operation unit according to the embodiment.
FIG. 7 is a diagram for illustrating translational movement of the robot arm.
FIG. 8 is a diagram for illustrating rotational movement of the robot arm.
FIG. 9 is a diagram showing an endoscope.
FIG. 10 is a diagram showing a pivot position setting instrument.
FIG. 11 is a diagram showing operation units according to the embodiment.
FIG. 12 is a diagram showing a right-handed wrist according to the embodiment.
FIG. 13 is a diagram showing a left-handed wrist according to the embodiment.
FIG. 14 is a perspective view showing foot pedals according to the embodiment.
FIG. 15 is a control block diagram of the robotic surgical system according to the embodiment.
FIG. 16 is a control block diagram of the robot arm according to the embodiment.
FIG. 17 is a control block diagram of a positioner and the medial cart according to the embodiment.
FIG. 18 is a control block diagram of the operation unit according to the embodiment.
FIG. 19 is a diagram for illustrating plane setting according to the embodiment.
FIG. 20 is a graph for illustrating a position coefficient according to the embodiment.
FIG. 21 is a graph for illustrating a speed coefficient when the operation unit is moved in a direction opposite to a protruding direction according to the embodiment.
FIG. 22 is a graph for illustrating a speed coefficient when the operation unit is moved in the protruding direction according to the embodiment.
FIG. 23 is a graph for illustrating limiting the amount of increase or decrease in the speed coefficient according to the embodiment.
FIG. 24 is a graph for illustrating a plane disappearance coefficient according to the embodiment.

### DESCRIPTION OF THE PREFERRED EMBODIMENTS

### Configuration of Robotic Surgical System

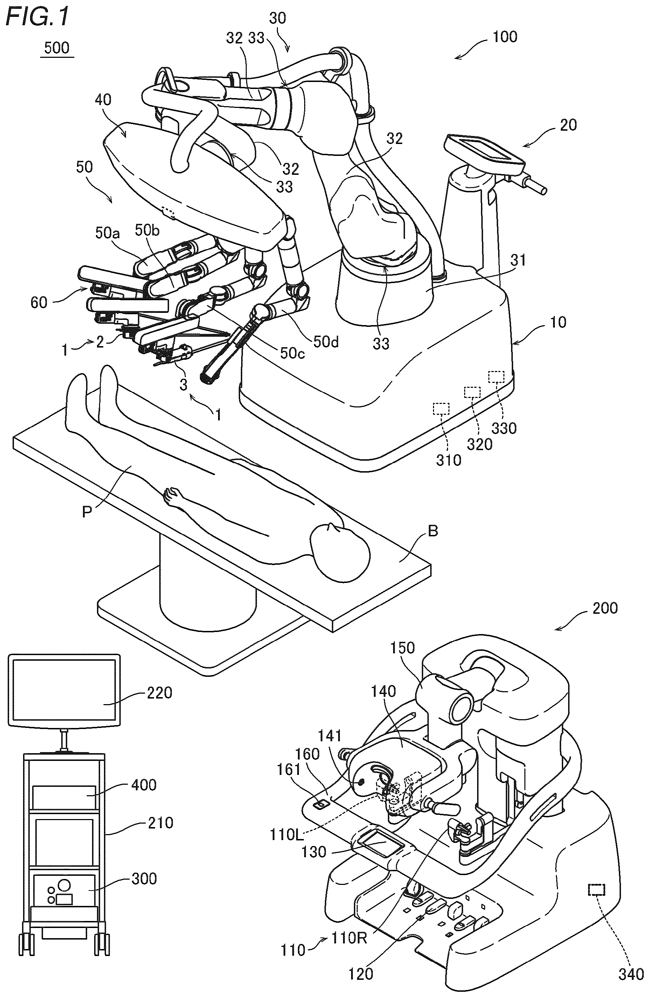

The configuration of a robotic surgical system 500 according to an embodiment is now described. As shown in FIG. 1, the robotic surgical system 500 includes a surgical robot 100, a remote control apparatus 200, a vision unit 300, and an image processing unit 400. The remote control apparatus 200 is an example of an operation apparatus.

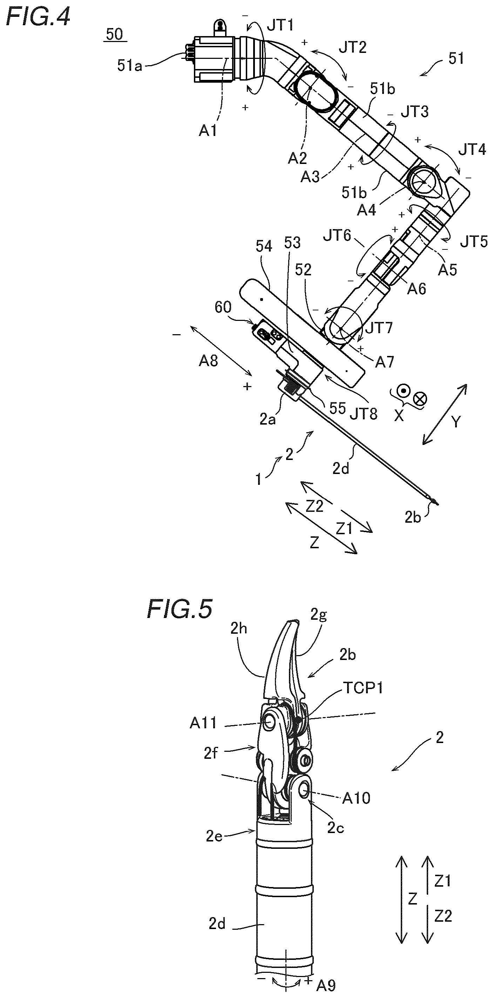

In this specification, as shown in FIG. 4, the longitudinal direction of a surgical instrument 1 is defined as a Z direction. The distal end side of the surgical instrument 1 is defined as a Z1 side, and the proximal end side of the surgical instrument 1 is defined as a Z2 side. A direction perpendicular to the Z direction is defined as an X direction. A direction perpendicular to the Z direction and the X direction is defined as a Y direction.

As shown in FIG. 1, the surgical robot 100 is arranged in an operating room. The remote control apparatus 200 is spaced apart from the surgical robot 100. The remote control apparatus 200 receives operations for the surgical instrument 1. Specifically, an operator such as a doctor inputs a command to the remote control apparatus 200 to cause the surgical robot 100 to perform a desired operation. The remote control apparatus 200 transmits the input command to the surgical robot 100. The surgical robot 100 operates based on the received command. The surgical robot 100 is arranged in the operating room that is a sterilized sterile field.

### Configuration of Surgical Robot

As shown in FIG. 1, the surgical robot 100 includes a medical cart 10, a cart positioner operation unit 20, a positioner 30, an arm base 40, a plurality of robot arms 50, and an arm operation unit 60 provided on each of the robot arms 50.

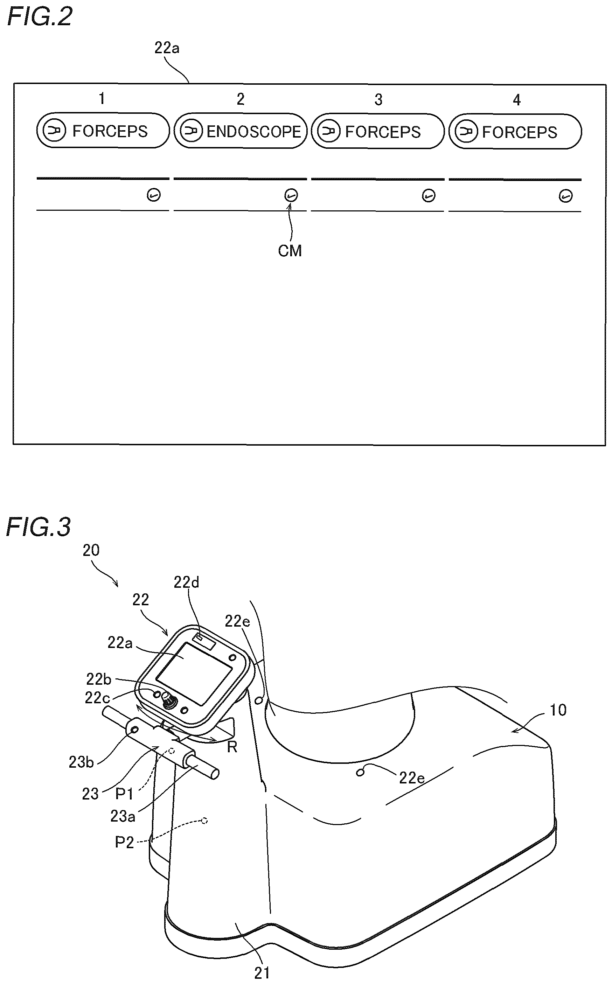

As shown in FIG. 3, the cart positioner operation unit 20 is supported by a cart positioner operation support 21 at the rear of the medical cart 10, and the medical cart 10 or the positioner 30 is moved by operating the cart positioner operation unit 20. The cart positioner operation unit 20 includes an input 22 and an operation handle 23. The input 22 receives operations to move the positioner 30, the arm base 40, and the plurality of robot arms 50 or change their postures mainly in order to prepare for surgery before the surgery.

As shown in FIG. 3, the input 22 includes a display 22a, a joystick 22b, an enable switch 22c, an error reset button 22d, and speakers 22e. The display 22a is a liquid crystal panel, for example. As shown in FIG. 2, the display 22a displays numbers corresponding to the plurality of robot arms 50. The display 22a also displays the type of surgical instrument 1 attached to each of the plurality of robot arms 50. A check mark CM indicating that a pivot position PP described below has been set is displayed on the display 22a.

As shown in FIG. 3, the joystick 22b is arranged in the vicinity of or adjacent to the display 22a of the input 22. The positioner 30 is moved three-dimensionally by selecting an operation mode displayed on the display 22a and operating the joystick 22b.

The enable switch 22c is arranged in the vicinity of or adjacent to the joystick 22b. The enable switch 22c enables or disables movement of the positioner 30. When the joystick 22b is operated while the enable switch 22c is pressed to enable movement of the positioner 30, the positioner 30 is moved.

The error reset button 22d resets errors in the robotic surgical system 500. The errors may be abnormal deviation errors, for example. The speakers 22e are arranged in pair. The pair of speakers 22e are arranged in the vicinity of or adjacent to the location of the positioner 30 in the medical cart 10.

The operation handle 23 is arranged in the vicinity of the display 22a. The operation handle 23 includes a throttle 23a that is gripped and twisted by an operator such as a nurse or a technician to operate movement of the medical cart 10. Specifically, the operation handle 23 is arranged below the input 22. As the throttle 23a is twisted from the near side to the far side, the medical cart 10 moves forward. As the throttle 23a is twisted from the far side to the near side, the medical cart 10 moves rearward. The speed of the medical cart 10 is changed according to a twisting amount of the throttle 23a. The operation handle 23 is rotatable to the left and right shown by an R direction, and the medical cart 10 is turned with rotation of the operation handle 23.

An enable switch 23b for enabling or disabling movement of the medical cart 10 is provided on the operation handle 23 of the cart positioner operation unit 20. When the throttle 23a of the operation handle 23 is operated while the enable switch 23b is pressed to enable movement of the medical cart 10, the medical cart 10 is moved.

As shown in FIG. 1, the positioner 30 includes a 7-axis articulated robot, for example. The positioner 30 is arranged on the medical cart 10. The positioner 30 adjusts the position of the arm base 40. The positioner 30 moves the position of the arm base 40 three-dimensionally.

The positioner 30 includes a base 31 and a plurality of links 32 coupled to the base 31. The plurality of links 32 are coupled to each other by joints 33.

The arm base 40 is attached to the distal end of the positioner 30. The proximal end of each of the plurality of robot arms 50 is attached to the arm base 40. Each of the plurality of robot arms 50 is able to take a folded and stored posture. The arm base 40 and the plurality of robot arms 50 are covered with sterile drapes and used. Moreover, each of the robot arms 50 supports the surgical instrument 1.

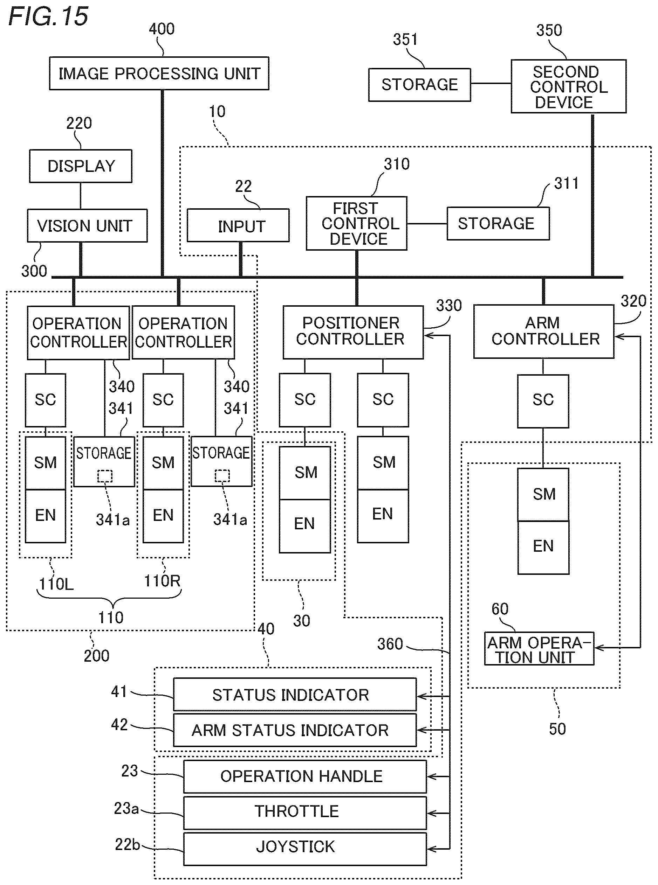

A status indicator 41 and an arm status indicator 42 that are shown in FIG. 15 are provided on the arm base 40. The status indicator 41 indicates the status of the robotic surgical system 500. The arm status indicator 42 indicates the statuses of the robot arms 50.

The plurality of robot arms 50 are arranged. Specifically, four robot arms 50a, 50b, 50c, and 50d are arranged. The robot arms 50a, 50b, 50c, and 50d have the same or similar configurations as each other.

As shown in FIG. 4, each robot arm 50 includes an arm portion 51, a first link 52, a second link 53, and a translation mechanism 54. The robot arm 50 includes joints JT1, JT2, JT3, JT4, JT5, JT6, JT7, and JT8. The joints JT1, JT2, JT3, JT4, JT5, JT6, and JT7 have A1, A2, A3, A4, A5, A6, and A7 axes as rotation axes, respectively. The joint JT8 has an A8 axis as a linear motion axis. The arm portion 51 includes a base 51a and links 51b.

The arm portion 51 includes a 7-axis articulated robot arm. The first link 52 is arranged at the distal end of the arm portion 51. The arm operation unit 60 described below is attached to the second link 53. The translation mechanism 54 is arranged between the first link 52 and the second link 53. A holder 55 that holds the surgical instrument 1 is arranged on the second link 53. The translation mechanism 54 translates the holder 55 to which the surgical instrument 1 is attached between a first position and a second position. The first position refers to an end position on the Z2 side in a range of movement of the holder 55 along the A8 axis by the translation mechanism 54. The second position refers to an end position on the Z1 side in the range of movement of the holder 55 along the A8 axis by the translation mechanism 54.

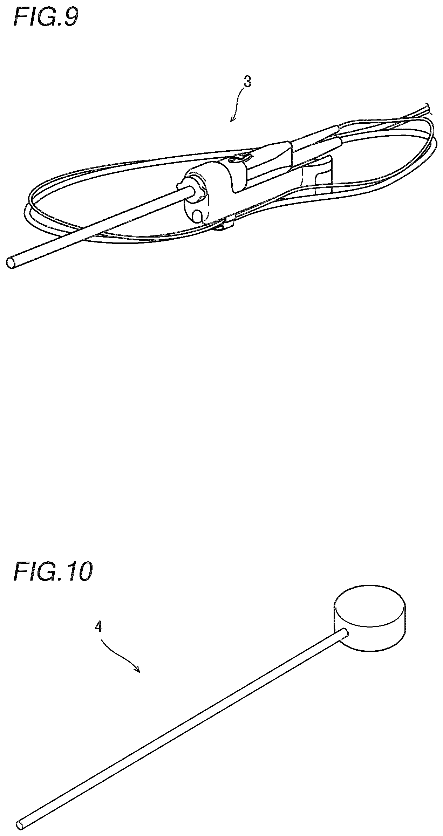

The surgical instrument 1 is attached to the distal end of each of the plurality of robot arms 50. The surgical instrument 1 includes a replaceable instrument 2, an endoscope 3 shown in FIG. 9 to capture an image of a surgical site, and a pivot position setting instrument 4 shown in FIG. 10 to set the pivot position PP, for example. The instrument 2 includes a driven unit 2a, an end effector 2b, a wrist joint 2c shown in FIG. 15, and a shaft 2d. The end effector 2b is connected to the distal end of the shaft 2d via the wrist joint 2c.

As shown in FIG. 1, the endoscope 3 is attached to the distal end of one of the plurality of robot arms 50, such as the robot arm 50c, and the instrument 2 is attached to the distal end of each of the remaining robot arms 50a, 50b, and 50d, for example. The endoscope 3 is preferably attached to one of two robot arms 50b and 50c arranged in the center among the four robot arms 50 arranged adjacent to each other.

### Configuration of Instrument

As shown in FIG. 5, the end effector 2b including jaw members 2g and 2h is attached to the distal end of the instrument 2, for example. As the end effector 2b, a pair of scissors, a grasper, a needle holder, a microdissector, a stable applier, a tacker, a suction cleaning tool, a snare wire, a clip applier, etc. can be used.

The instrument 2 includes a first support member 2e and a second support member 2f. The first support member 2e is attached to the shaft 2d. The second support member 2f is supported by the first support member 2e so as to be rotatable around an A10 axis, and supports the end effector 2b such that the end effector 2b is rotatable around an A11 axis that intersects with the A10 axis. The shaft 2d rotates around an A9 axis. The wrist joint 2c is provided between the second support member 2f and the first support member 2e with the A10 axis as a rotation axis.

### Configuration of Arm Operation Unit

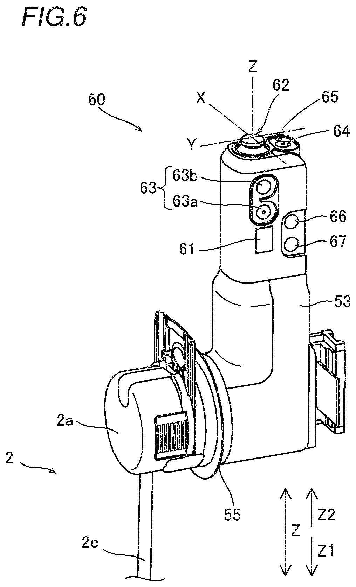

As shown in FIG. 6, the arm operation unit 60 is attached to the robot arm 50 to operate the robot arm 50. Specifically, the arm operation unit 60 is attached to the second link 53.

The arm operation unit 60 includes an enable switch 61, a joystick 62, and linear switches 63, a mode switching button 64, a mode indicator 65, a pivot button 66, and an adjustment button 67.

The enable switch 61 is pressed to enable or disable movement of the robot arm 50 in response to the joystick 62 and the linear switches 63. When the enable switch 61 is pressed by an operator such as a nurse or an assistant grasping the arm operation unit 60, movement of the surgical instrument 1 by the robot arm 50 is enabled.

The joystick 62 is an operation tool to control movement of the surgical instrument 1 by the robot arm 50. The joystick 62 controls a moving direction and a moving speed of the robot arm 50. The robot arm 50 is moved in accordance with a tilting direction and a tilting angle of the joystick 62.

The linear switches 63 are switches to move the surgical instrument 1 in the Z direction, which is the longitudinal direction of the surgical instrument 1. The linear switches 63 include a linear switch 63a to move the surgical instrument 1 in a direction in which the surgical instrument 1 is inserted into a patient P, and a linear switch 63b to move the surgical instrument 1 in a direction in which the surgical instrument 1 is moved away from the patient P. Both the linear switch 63a and the linear switch 63b are push-button switches.

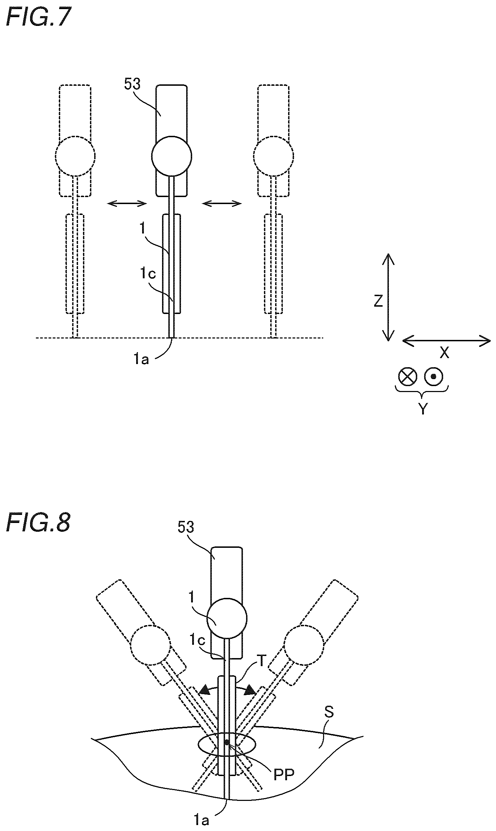

The mode switching button 64 is a push-button switch to switch between a mode for translationally moving the surgical instrument 1 and a mode for rotationally moving the surgical instrument 1. As shown in FIG. 7, in the mode for translationally moving the robot arm 50, the robot arm 50 is moved such that the distal end 1a of the surgical instrument 1 is moved in an X-Y plane. As shown in FIG. 8, in the mode for rotationally moving the robot arm 50, the robot arm 50 is moved such that the surgical instrument 1 is rotationally moved around, as a fulcrum, a center on the A11 axis of the end effector 2b or the distal end of the end effector 2b of the instrument 2 serving as the surgical instrument 1 when any pivot position PP is not stored in a storage 351, and the surgical instrument 1 is rotationally moved around the pivot position PP as a fulcrum when the pivot position PP is stored in the storage 351. In this case, the surgical instrument 1 is rotationally moved with the shaft 1c of the surgical instrument 1 inserted into a trocar T and the distal end 1a of the surgical instrument 1 inserted into the body surface S of the patient P. The mode switching button 64 is arranged on a Z-direction side surface of the arm operation unit 60.

The mode indicator 65 indicates a switched mode. The mode indicator 65 is on to indicate a rotational movement mode and is off to indicate a translational movement mode. Furthermore, the mode indicator 65 also serves as a pivot position indicator that indicates that the pivot position PP has been set. The mode indicator 65 is arranged on the Z-direction side surface of the arm operation unit 60.

The pivot button 66 is a push-button switch to set the pivot position PP that serves as a fulcrum for movement of the surgical instrument 1 attached to the robot arm 50.

The adjustment button 67 is a button to optimize the position of the robot arm 50. After the pivot position PP for the robot arm 50 to which the endoscope 3 has been attached is set, the positions of the other robot arms 50 and the arm base 40 are optimized when the adjustment button 67 is pressed. The adjustment button 67 is a button different from the enable switch 61.

### Remote Control Apparatus

As shown in FIG. 1, the remote control apparatus 200 is arranged inside or outside the operating room, for example. The remote control apparatus 200 includes operation units 110, foot pedals 120, a touch panel 130, a monitor 140, a support arm 150, a support bar 160, and an error reset button 161. The operation units 110 include operation handles for the operator such as a doctor to input a command.

### Operation Unit

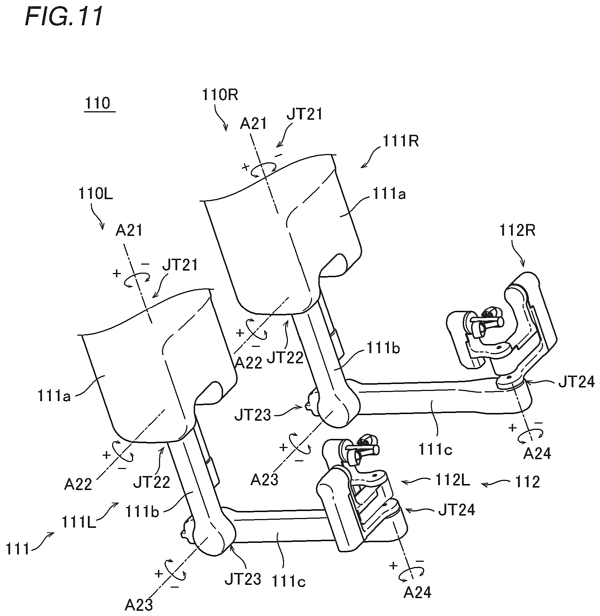

As shown in FIG. 11, the operation units 110 each include a handle to operate the surgical instrument 1. The operation unit 110 receives an operation for the surgical instrument 1. The operation units 110 include an operation unit 110L that is located on the left side as viewed from the operator such as a doctor and is to be operated by the left hand of the operator, and an operation unit 110R that is located on the right side and is to be operated by the right hand of the operator. The operation units 110 include arms 111 and wrists 112. The operation unit 110R includes an arm 111R and a wrist 112R. The operation unit 110L includes an arm 111L and a wrist 112L.

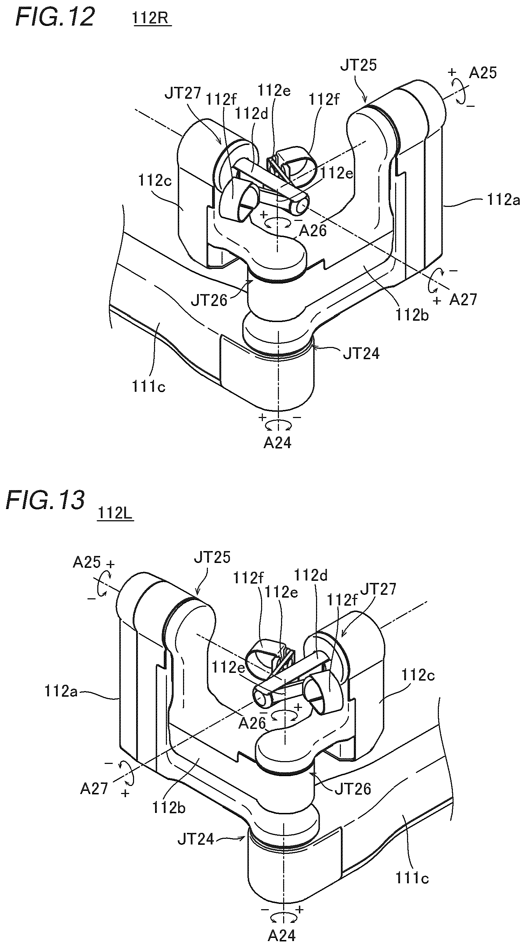

The arms 111 each have joints JT21, JT22, and JT23 shown in FIG. 11, and joints JT24, JT25, JT26, and JT27 shown in FIGS. 12 and 13. The rotation axes of the joints JT21, JT22, JT23, JT24, JT25, JT26, and JT27 are A21, A22, A23, A24, A25, A26, and A27 axes, respectively.

### Arm

As shown in FIG. 11, the arm 111R includes a link 111a, a link 111b, and a link 111c. The upper end side of the link 111a is attached to the remote control apparatus 200 such that the link 111a is rotatable around the A21 axis along a vertical direction. The upper end side of the link 111b is attached to the lower end side of the link 111a such that the link 111b is rotatable around the A22 axis along a horizontal direction. A first end side of the link 111c is attached to the lower end side of the link 111b such that the link 111c is rotatable around the A23 axis along the horizontal direction. The wrist 112 is attached to a second end side of the link 111c such that the wrist 112 is rotatable around the A24 axis. The link 111a is connected to the remote control apparatus 200 by the joint JT21. The link 111a and the link 111b are connected to each other by the joint JT22. The link 111b and the link 111c are connected to each other by the joint JT23. The arm 111 supports the wrist 112. The arm 111L has the same or similar configuration as the arm 111R.

The wrists 112 include the wrist 112R shown in FIG. 12 and to be operated by the right hand of the operator, and the wrist 112L shown in FIG. 13 and to be operated by the left hand of the operator. FIG. 12 shows the reference posture of the operation unit 110R, and FIG. 13 shows the reference posture of the operation unit 110L. The configuration of the wrist 112R is the same as or similar to that of the wrist 112L.

The wrists 112 each include a link 112a, a link 112b, a link 112c, and a grip support member 112d that is to be operated by the operator such as a doctor. The link 112a includes a proximal end connected to the distal end of the arm 111 and rotates around the A24 axis. The link 112b includes a proximal end connected to the distal end of the link 112a and rotates around the A25 axis with respect to the link 112a. The link 112c includes a proximal end connected to the distal end of the link 112b and a distal end to which the grip support member 112d is connected, and rotates around the A26 axis with respect to the link 112b. The grip support member 112d rotates around the A27 axis with respect to the link 112c. The link 112a, the link 112b, and the link 112c each have an L shape.

The wrist 112 includes a pair of grip members 112e that are opened and closed by the operator. The grip members 112e each include an elongated plate-shaped lever member, and the proximal ends of the pair of grip members 112e are rotatably connected to the proximal end of the grip support member 112d. Cylindrical finger insertion portions 112f are provided on the grip members 112e. The operator inserts their fingers into a pair of finger insertion portions 112f to operate the wrist 112. The proximal ends of the pair of grip members 112e are connected to the grip support member 112d, and an angle between the pair of grip members 112e is increased or decreased such that an opening angle between the jaw member 2g and the jaw member 2h is changed. A magnet is provided on one of the grip members 112e, and a Hall sensor is provided on the grip support member 112d. When the operator opens and closes the grip members 112e, the magnet and the Hall sensor function as an angle detection sensor, and the Hall sensor outputs the opening angle. As the angle detection sensor, the Hall sensor may be provided on the grip member 112e, and the magnet may be provided on the grip support member 112d. Alternatively, the magnet or the Hall sensor may be provided as the angle detection sensor on both the grip members 112e.

As shown in FIG. 1, the monitor 140 is a scope-type display that displays images captured by the endoscope 3. A notifier 141 is provided on the monitor 140. The notifier 141 issues an error sound. The support arm 150 supports the monitor 140 so as to align the height of the monitor 140 with the height of the face of the operator such as a doctor. The touch panel 130 is arranged on the support bar 160. The head of the operator is detected by a sensor provided in the vicinity of the monitor 140 such that the surgical robot 100 can be operated by the remote control apparatus 200. The operator operates the operation units 110 and the foot pedals 120 while visually recognizing an affected area on the monitor 140. Thus, a command is input to the remote control apparatus 200. The command input to the remote control apparatus 200 is transmitted to the surgical robot 100.

### Foot Pedals

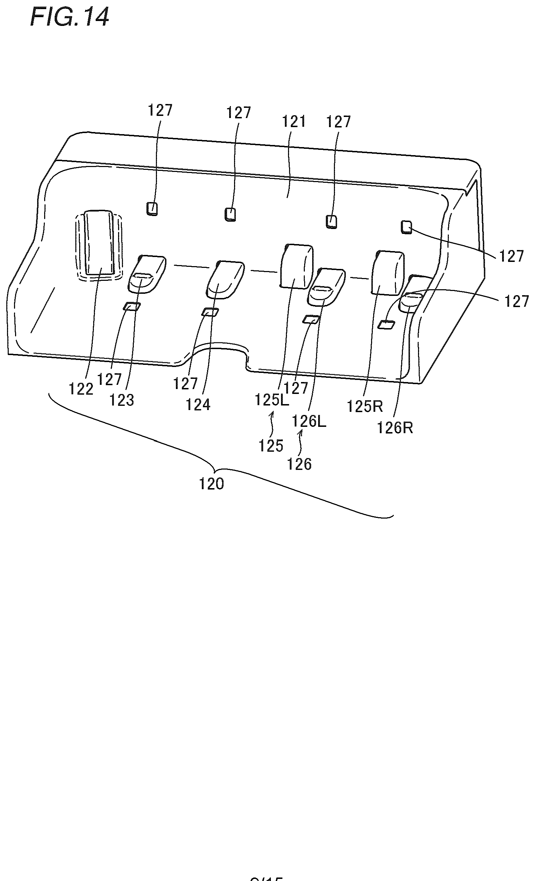

As shown in FIG. 14, a plurality of foot pedals 120 are provided to perform functions related to the surgical instrument 1. The plurality of foot pedals 120 are arranged on a base 121. The foot pedals 120 include a switching pedal 122, a clutch pedal 123, a camera pedal 124, incision pedals 125, coagulation pedals 126, and foot detectors 127. The switching pedal 122, the clutch pedal 123, the camera pedal 124, the incision pedals 125, and the coagulation pedals 126 are operated by the foot of the operator. The incision pedals 125 include an incision pedal 125R for a right robot arm 50, and an incision pedal 125L for a left robot arm 50. The coagulation pedals 126 include a coagulation pedal 126R for the right robot arm 50 and a coagulation pedal 126L for the left robot arm 50.

The switching pedal 122 switches robot arms 50 to be operated by the operation units 110. The clutch pedal 123 performs a clutch operation to temporarily disconnect an operation connection between the robot arms 50 and the operation units 110. While the clutch pedal 123 is being pressed by the operator, operations by the operation units 110 are not transmitted to the robot arms 50. While the camera pedal 124 is being pressed by the operator, the operation unit 110 can operate a robot arm 50 to which the endoscope 3 is attached. While the incision pedals 125 or the coagulation pedals 126 are being pressed by the operator, an electrosurgical device is activated.

### Vision Unit and Image Processing Unit

As shown in FIG. 1, the vision unit 300 and the image processing unit 400 are placed on a cart 210. The image processing unit 400 processes images captured by the endoscope 3. A display 220 is arranged on the cart 210. The display 220 displays images captured by the endoscope 3.

### Configuration of Control System

As shown in FIG. 15, the robotic surgical system 500 includes a first control device 310, an arm controller 320, a positioner controller 330, operation controllers 340, and a second control device 350. The robotic surgical system 500 also includes a storage 311 connected to the first control device 310, storages 341 connected to the operation controllers 340, and the storage 351 connected to the second control device 350. The operation controllers 340 include computers including processors such as CPUs, and executes various processes by executing programs 341a. The programs 341a are stored in the storages 341, for example. The operation controllers 340 are examples of a control device, a controller, or a computer.

The first control device 310 is accommodated in the medical cart 10 to communicate with the arm controller 320 and the positioner controller 330, and controls the entire robotic surgical system 500. Specifically, the first control device 310 communicates with and controls the arm controller 320, the positioner controller 330, and the operation controllers 340. The first control device 310 is connected to the arm controller 320, the positioner controller 330, and the operation controllers 340 through a LAN, for example. The first control device 310 is arranged inside the medical cart 10.

The arm controller 320 is arranged for each of the plurality of robot arms 50. That is, the same number of arm controllers 320 as the plurality of robot arms 50 are placed inside the medical cart 10.

As shown in FIG. 15, the input 22 is connected to the first control device 310 through a LAN, for example. The status indicator 41, the arm status indicator 42, the operation handle 23, the throttle 23a, and the joystick 22b are connected to the positioner controller 330 via a wire line 360 by means of a communication network that allows information to be shared with each other by using serial communication. Although FIG. 15 shows that the status indicator 41, the arm status indicator 42, etc. are all connected to one wire line 360, in reality, the wire line 360 is arranged for each of the status indicator 41, the arm status indicator 42, the operation handle 23, the throttle 23a, and the joystick 22b.

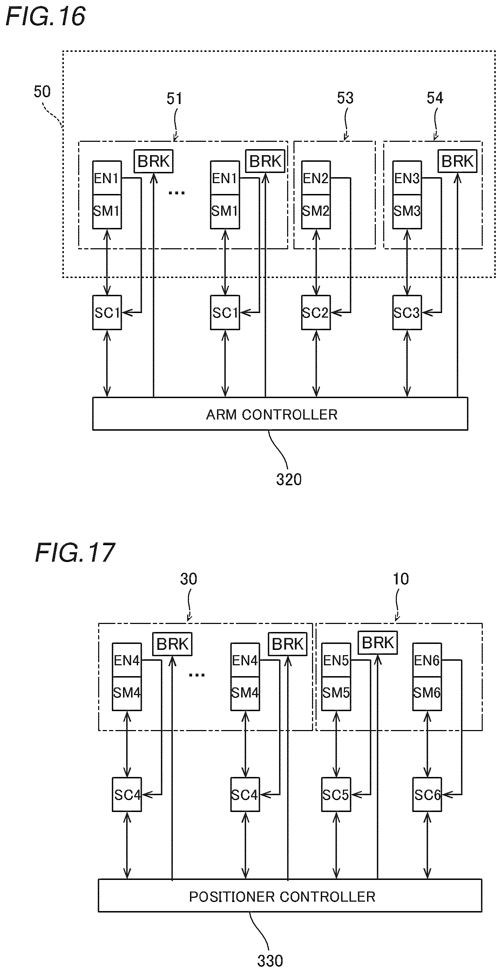

As shown in FIG. 16, the arm portion 51 includes a plurality of servomotors SM1, encoders EN1, and speed reducers so as to correspond to the joints JT1, JT2, JT3, JT4, JT5, JT6, and JT7. The encoders EN1 detect rotation angles of the servomotors SM1. The speed reducers slow down rotation of the servomotors SM1 to increase the torques. Inside the medical cart 10, servo controllers SC1 that control the servomotors SM1 are arranged adjacent to the arm controller 320. In addition, the encoders EN1 that detect the rotation angles of the servomotors SM1 are electrically connected to the servo controllers SC1.

Servomotors SM2 that rotate driven members provided in the driven unit 2a of the surgical instrument 1, encoders EN2, and speed reducers are arranged in the second link 53. The encoders EN2 detect rotation angles of the servomotors SM2. The speed reducers slow down rotation of the servomotors SM2 to increase the torques. In the medical cart 10, servo controllers SC2 are provided to control the servomotors SM2 to drive the surgical instrument 1. The encoders EN2 that detect the rotation angles of the servomotors SM2 are electrically connected to the servo controllers SC2. A plurality of servomotors SM2, a plurality of encoders EN2, and a plurality of servo controllers SC2 are arranged.

The translation mechanism 54 includes a servomotor SM3 to translationally move the surgical instrument 1, an encoder EN3, and a speed reducer. The encoder EN3 detects a rotation angle of the servomotor SM3. The speed reducer slows down rotation of the servomotor SM3 to increase the torque. In the medical cart 10, a servo controller SC3 is provided to control the servomotor SM3 to translationally move the surgical instrument 1. The encoder EN3 that detects the rotation angle of the servomotor SM3 is electrically connected to the servo controller SC3.

The first control device 310 generates command values to command the positions of the servomotors SM1, SM2, and SM3 based on operations received by the remote control apparatus 200, and drives the servomotors SM1, SM2, and SM3 based on the command values. The first control device 310 detects an abnormal deviation error when differences between the command values and the positions of the servomotors SM1, SM2, and SM3 detected by sensors exceed an allowable range.

As shown in FIG. 17, a plurality of servomotors SM4, a plurality of encoders EN4, and a plurality of speed reducers are provided in the positioner 30 so as to correspond to the plurality of joints 33 of the positioner 30. The encoders EN4 detect rotation angles of the servomotors SM4. The speed reducers slow down rotation of the servomotors SM4 to increase the torques.

The medical cart 10 includes wheels including front wheels as drive wheels and rear wheels that are steered by the operation handle 23. The rear wheels are arranged closer to the operation handle 23 than the front wheels. The medical cart 10 includes servomotors SM5 to drive a plurality of front wheels of the medical cart 10, encoders EN5, speed reducers, and brakes BRK. The speed reducers slow down rotation of the servomotors SM5 to increase the torques. A potentiometer P1 shown in FIG. 3 is provided on the operation handle 23, and the servomotors SM5 of the front wheels are driven based on a rotation angle detected by the potentiometer P1 according to the twist of the throttle 23a. Rear wheels of the medical cart 10 are of the dual wheel type, and the rear wheels are steered based on rightward-leftward rotation of the operation handle 23. Furthermore, a potentiometer P2 shown in FIG. 3 is provided on a rotation axis of the operation handle 23, and servomotors SM6, encoders EN6, and speed reducers are provided on the rear wheels of the medical cart 10. The speed reducers slow down rotation of the servomotors SM6 to increase the torques. The servomotors SM6 are driven based on a rotation angle detected by the potentiometer P2 according to rightward-leftward rotation of the operation handle 23. That is, steering of the rear wheels by the rightward-leftward rotation of the operation handle 23 is power-assisted by the servomotors SM6.

The front wheels of the medical cart 10 are driven such that the medical cart 10 moves in the forward-rearward direction. Furthermore, the operation handle 23 is rotated such that the rear wheels are steered, and the medical cart 10 turns in a right-left direction.

As shown in FIG. 17, in the medical cart 10, servo controllers SC4 are provided to control the servomotors SM4 to move the positioner 30. The encoders EN4 that detect the rotation angles of the servomotors SM4 are electrically connected to the servo controllers SC4. In the medical cart 10, servo controllers SC5 are provided to control the servomotors SM5 to drive the front wheels of the medical cart 10. The encoders EN5 that detect the rotation angles of the servomotors SM5 are electrically connected to the servo controllers SC5. In the medical cart 10, servo controllers SC6 are provided to control the servomotors SM6 to power-assist steering of the rear wheels of the medical cart 10. The encoders EN6 that detect the rotation angles of the servomotors SM6 are electrically connected to the servo controllers SC6.

As shown in FIGS. 16 and 17, the joints JT1, JT2, JT3, JT4, JT5, JT6, and JT7 of the arm portion 51, and the joints 33 of the positioner 30 include brakes BRK. Furthermore, the front wheels of the medical cart 10, the arm base 40, and the translation mechanism 54 include brakes BRK. The arm controller 320 unidirectionally transmits control signals to the brakes BRK of the joints JT1, JT2, JT3, JT4, JT5, JT6, and JT7 of the arm portion 51 and the translation mechanism 54. The control signals are signals for turning on/off the brakes BRK. The signals for turning on the brakes BRK include signals for maintaining the brakes BRK in an enabled state. The same applies to control signals from the positioner controller 330 to the brakes BRK of the joints 33 of the positioner 30 and the arm base 40. On startup, all the brakes BRK of the arm base 40, the arm portion 51, and the translation mechanism 54 are released but the servomotors SM are driven against gravity to maintain the postures of the robot arm 50 and the arm base 40. When an error occurs in the robotic surgical system 500, the brakes BRK of the arm base 40, the arm portion 51 and the translation mechanism 54 are turned on. When the error in the robotic surgical system 500 is reset, the brakes BRK of the arm base 40, the arm portion 51, and the translation mechanism 54 are turned off. When a shutdown operation is performed in the robotic surgical system 500, the brakes BRK of the arm base 40, the arm portion 51, and the translation mechanism 54 are turned on. The brakes BRK of the front wheels of the medical cart 10 are constantly turned on, and the brakes BRK are deactivated only while the enable switch 23b is being pressed. The brakes BRK of the joints 33 of the positioner 30 are constantly turned on, and the brakes BRK are deactivated only while the enable switch 22c is being pressed.

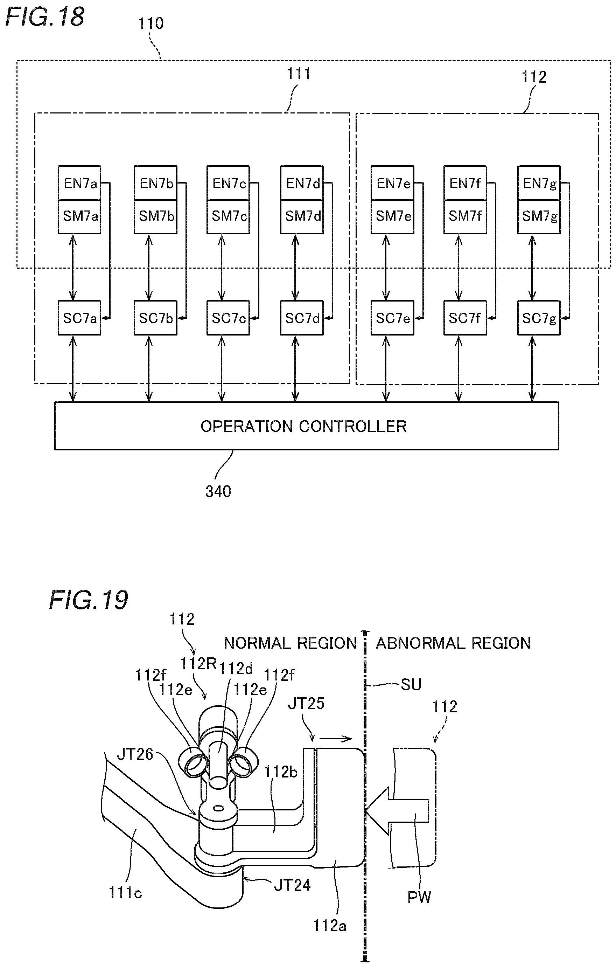

As shown in FIG. 18, servomotors SM7a, SM7b, SM7c, SM7d, SM7e, SM7f, and SM7g are arranged on the joints JT21, JT22, JT23, JT24, JT25, JT26, and JT27 of the operation unit 110, respectively. The servomotor SM7a rotates the link 111a around the A21 axis. The servomotor SM7b rotates the link 111b around the A22 axis. The servomotor SM7c rotates the link 111c around the A23 axis. The servomotor SM7d rotates the link 112a around the A24 axis. The servomotor SM7e rotates the link 112b around the A25 axis. The servomotor SM7f rotates the link 112c around the A26 axis. The servomotor SM7g rotates the grip support member 112d around the A27 axis. Servo controllers SC7a, SC7b, SC7c, SC7d, SC7e, SC7f, and SC7g are provided to control the servomotors. Encoders EN7a, EN7b, EN7c, EN7d, EN7e, EN7f, and EN7g are electrically connected to the servo controllers to detect rotation angles of the servomotors. The servomotors, the servo controllers, and the encoders are provided in each of the operation unit 110L and the operation unit 110R. The servomotors SM7a, SM7b, SM7c, SM7d, SM7e, SM7f, and SM7g are provided to assist the operator in performing an operation. The servomotors SM7a, SM7b, and SM7c are examples of a drive.

The first control device 310 controls the servomotors via the operation controllers 340 to generate torques that counteract gravitational torques generated on rotation axes of the servomotors according to the postures of the operation units 110. Thus, the operator can operate the operation units 110 with a relatively small force.

When the operator performs an operation to rotate the grip support member 112d around the A27 axis of the operation unit 110 shown in FIGS. 12 and 13, the shaft 2d of the instrument 2 rotates around the A9 axis shown in FIG. 5. When the operator performs an operation to rotate the joints JT24, JT25, and JT26 of the operation unit 110 shown in FIGS. 12 and 13, the end effector 2b bends around the A10 axis or the A11 axis shown in FIG. 5.

The operation controllers 340 are arranged in a main body of the remote control apparatus 200. The operation controllers 340 control the operation units 110. The operation controllers 340 are provided so as to correspond to the left-handed operation unit 110L and the right-handed operation unit 110R, respectively, as shown in FIG. 15.

As shown in FIG. 15, the vision unit 300 and the image processing unit 400 are connected to the first control device 310 through a LAN, for example. The display 220 is connected to the vision unit 300.

### Plane Setting

In this embodiment, as shown in FIG. 19, when the operation unit 110 is moved such that the robot arm 50 is attempting to move out of a normal region, the operation controller 340 sets a plane SU, and when the operation unit 110 crosses the plane SU, the operation controller 340 controls at least one of a plurality of servomotors SM7a, SM7b, SM7c, SM7d, SM7e, SM7f, and SM7g to apply a force PW to the operation unit 110. Specifically, the operation controller 340 performs a control to apply the force PW by controlling the servomotors SM7a, SM7b, and SM7c of the joints JT21, JT22, and JT23, which are positioning joints, among a plurality of joints JT21, JT22, JT23, JT24, JT25, JT26, and JT27. For convenience, the operation unit 110R is illustrated in FIG. 19, but the same applies to the operation unit 110L.

In this embodiment, the operation controller 340 determines the operation direction of the operation unit 110 in which the robot arm 50 returns to the normal region, and controls the servomotors SM7a, SM7b, and SM7c to apply the force PW in the determined operation direction. That is, when the operation unit 110 crosses the plane SU, the operation controller 340 controls the servomotors SM7a, SM7b, and SM7c to apply the force PW in a direction in which the operation unit 110 is pushed back from the plane SU. Even when the operation unit 110 moves parallel to the plane SU, the force PW acts in the same direction.

In this embodiment, the plane SU is flat. The plane SU is set based on boundary points between the normal region and an abnormal region outside the normal region in the coordinate system of the operation unit 110. The operation controller 340 performs a control to acquire the boundary points between the normal region and the abnormal region in the coordinate system of the operation unit 110 based on the state of the robot arm 50. Then, the operation controller 340 performs a control to acquire a plane including the boundary points between the normal region and the abnormal region in the coordinate system of the operation unit 110 by calculation, and set the acquired plane as the plane SU.

In this embodiment, the operation controller 340 performs a control to switch setting of the plane SU between enabled and disabled based on a predetermined condition. The predetermined condition for switching setting of the plane SU to enabled includes a case in which a restriction is imposed on movement of the robot arm 50. For example, cases in which a restriction is imposed on movement of the robot arm 50 include a case in which interference occurs between the robot arms 50, a case in which a restriction is imposed due to the pivot position PP, a case in which a joint range of motion of the robot arm 50 is restricted, a case in which an inverse transformation error occurs in a movement command for the robot arm 50, and a case in which a movement restriction is imposed due to contact detection.

When interference occurs between the robot arms 50, a restriction is imposed on movement of the robot arms 50 such that the interfering robot arms 50 do not approach each other any closer than the current distance. In such a case, the normal region is a region in which the interference between the robot arms 50 is resolved, and the abnormal region is a region in which the interference between the robot arms 50 occurs.

The restriction due to the pivot position PP refers to a restriction that prevents the distal end 1a of the surgical instrument 1 from moving in a direction away from the patient P with respect to the pivot position PP. When a restriction due to the pivot position PP is imposed, a restriction is imposed on movement of the robot arm 50 such that the distal end 1a of the surgical instrument 1 does not move in the direction away from the patient P with respect to the pivot position PP. In such a case, the normal region is a region in which the distal end 1a of the surgical instrument 1 is moved closer to the patient P, and the abnormal region is a region in which the distal end 1a of the surgical instrument 1 is moved farther away from the patient P.

The restriction on the joint range of motion of the robot arm 50 refers to a restriction that prevents the joint rotation angle of the robot arm 50 from going outside the range of the software limit. When a restriction on the joint range of motion of the robot arm 50 is imposed, a restriction on movement of the robot arm 50 is imposed such that the joint rotation angle of the robot arm 50 does not go outside the range of the software limit. In such a case, the normal region is a region in which the joint rotation angle of the robot arm 50 is within the range of the software limit, and the abnormal region is a region in which the joint rotation angle of the robot arm 50 is outside the range of the software limit.

An inverse transformation error in the movement command for the robot arm 50 occurs, for example, when the robot arm 50 is in a fully extended posture, and an error occurs because an inverse transformation process to acquire the movement command cannot be performed. When an inverse transformation error occurs in the movement command for the robot arm 50, a restriction is imposed on movement of the robot arm 50 such that the robot arm 50 does not take a posture in which an inverse transformation error occurs. In such a case, the normal region is a region in which the inverse transformation error is eliminated, and the abnormal region is a region in which the inverse transformation error occurs.

A movement restriction due to contact detection is a restriction that prevents the robot arm 50 from moving toward a contacted object when contact between the robot arm 50 and a surrounding object is detected due to a deviation abnormality, for example. When a movement restriction due to contact detection is imposed, movement of the robot arm 50 is restricted such that the robot arm 50 does not move toward the contacted object. In such a case, the normal region is a region in which the robot arm 50 is moved away from the contacted object, and the abnormal region is a region in which the robot arm 50 approaches the contacted object.

The predetermined condition for switching setting of the plane SU to disabled includes a case in which movement of the robot arm 50 is no longer restricted. Specifically, the predetermined condition for switching setting of the plane SU to disabled includes a case in which movement of the robot arm 50 is no longer restricted and a case in which the surgical instrument 1 is in a predetermined state. For example, in a case of an operation mode other than an operation mode in which the pivot position PP is set, when movement of the robot arm 50 is no longer restricted and the control point of the surgical instrument 1 or the attachment surface of the surgical instrument 1 to the robot arm 50 has moved a predetermined distance or more from a position at which setting of the plane SU was enabled, setting of the plane SU is switched to disabled. The predetermined distance is 10 mm, for example. Furthermore, for example, in a case of the operation mode in which the pivot position PP is set, setting of the plane SU is switched to disabled when movement of the robot arm 50 is no longer restricted and the distal end 1a of the surgical instrument 1 is inserted into the body surface S of the patient P by a predetermined insertion amount or more. The predetermined insertion amount is 10 mm, for example.

The predetermined condition for switching setting of the plane SU to disabled include a case in which movement of the robot arm 50 is no longer restricted, the surgical instrument 1 is in the predetermined state, and a predetermined period of time has elapsed in addition to a case in which movement of the robot arm 50 is no longer restricted and a case in which the surgical instrument 1 is in the predetermined state. The predetermined period of time is 200 ms, for example.

In this embodiment, the operation controller 340 performs a control to determine a position coefficient CP to adjust the magnitude of the force PW based on the protrusion amount AM of the operation unit 110 from the plane SU into the abnormal region. Specifically, when the protrusion amount AM is zero, the operation controller 340 sets the magnitude of the position coefficient CP to zero, and when the protrusion amount AM is greater than zero, the operation controller 340 increases the magnitude of the position coefficient CP as the protrusion amount AM increases. More specifically, when the protrusion amount AM is greater than zero and is equal to or less than a protrusion amount threshold ATH, the operation controller 340 increases the magnitude of the position coefficient CP as the protrusion amount AM increases, and when the protrusion amount AM is greater than the protrusion amount threshold ATH, the operation controller 340 maintains the magnitude of the position coefficient CP constant at a maximum value. When the position coefficient CP increases, the force PW for returning the operation unit 110 to the normal region increases.

The position coefficient CP has a minimum value of 0 and a maximum value of 1. The protrusion amount threshold ATH is 5 mm, for example. When the protrusion amount threshold ATH is too small, a relatively large force PW acts immediately after the operation unit 110 crosses the plane SU, and the operation unit 110 is prone to wobbling. On the other hand, when the protrusion amount threshold ATH is too large, the moving distance of the operation unit 110 from when movement of the robot arm 50 is restricted until when the operation unit 110 stops becomes large. Therefore, it is preferable to set the protrusion amount threshold ATH to a value that strikes a balance between these two, such as 5 mm.

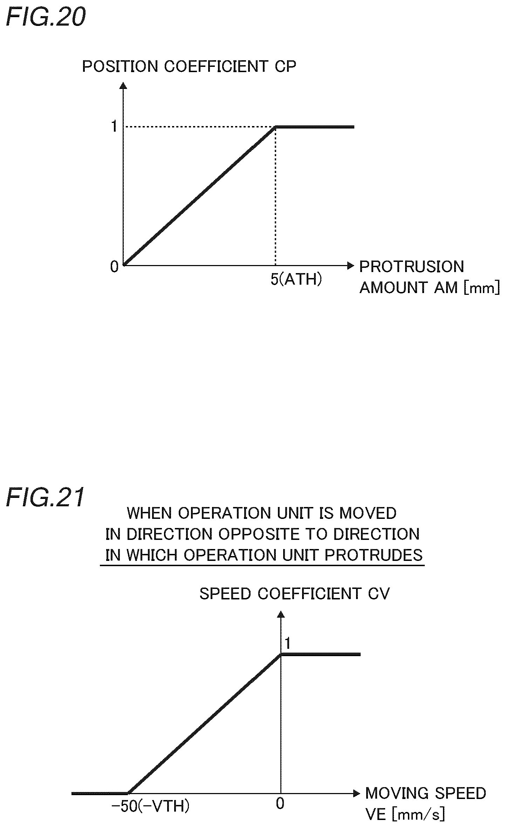

The operation controller 340 performs a control to acquire the protrusion amount AM based on the position of the plane SU, the current position of the operation unit 110, and the normal vector of the plane SU. Specifically, the operation controller 340 performs a control to acquire the protrusion amount AM by a formula AM = (P_{SU} - P_{H}) ·N_{SU}, where P_{SU} represents the position vector of the position of the plane SU, P_{H} represents the position vector of the current position of the operation unit 110, and N_{SU} represents the normal vector of the plane SU. The normal vector N_{SU} is a vector perpendicular to the plane SU. The operation controller 340 also performs a control to acquire the position coefficient CP corresponding to the acquired protrusion amount AM using correspondence information such as a table that associates the protrusion amount AM with the position coefficient CP, as shown in a graph of FIG. 20, for example, and determine it as a position coefficient CP to be used to adjust the magnitude of the force PW.

In this embodiment, as shown in FIG. 21, the operation controller 340 performs a control to determine a speed coefficient CV to adjust the magnitude of the force PW based on the moving speed VE of the operation unit 110. Specifically, when the operation unit 110 is moved in a direction opposite to a direction in which the operation unit 110 protrudes from the plane SU into the abnormal region, the operation controller 340 reduces the magnitude of the speed coefficient CV as the absolute value of the moving speed VE of the operation unit 110 increases when the absolute value of the moving speed VE of the operation unit 110 is greater than zero and equal to or less than a speed threshold VTH, and sets the magnitude of the speed coefficient CV to zero when the absolute value of the moving speed VE of the operation unit 110 is greater than the speed threshold VTH. When the operation unit 110 is moved in the direction in which the operation unit 110 protrudes from the plane SU into the abnormal region, the operation controller 340 sets the magnitude of the speed coefficient CV to 1 regardless of the absolute value of the moving speed VE of the operation unit 110. When the operation unit 110 is moved in the direction opposite to the direction in which the operation unit 110 protrudes from the plane SU into the abnormal region, the moving speed VE of the operation unit 110 is a negative value. When the operation unit 110 is moved in the direction in which the operation unit 110 protrudes from the plane SU into the abnormal region, the moving speed VE of the operation unit 110 is a positive value. The speed coefficient CV has a minimum value of 0 and a maximum value of 1. The speed threshold VTH is 50 mm/s, for example.

The operation controller 340 performs a control to acquire the moving speed VE of the operation unit 110 based on the protrusion amount AM of the current control cycle, the protrusion amount AM of the previous control cycle, and the time of one control cycle. Specifically, the operation controller 340 performs a control to acquire the moving speed VE of the operation unit 110 by a formula VE = (Amₛ - AM_{old}) /T_{cycle}, where Amₛ represents the protrusion amount AM of the current control cycle, Am_{old} represents the protrusion amount AM of the previous control cycle, and T_{cycle} represents the time of one control cycle. The operation controller 340 also performs a control to acquire the speed coefficient CV corresponding to the acquired moving speed VE of the operation unit 110 using correspondence information such as a table that associates the moving speed VE of the operation unit 110 with the speed coefficient CV, as shown in a graph of FIG. 21, for example.

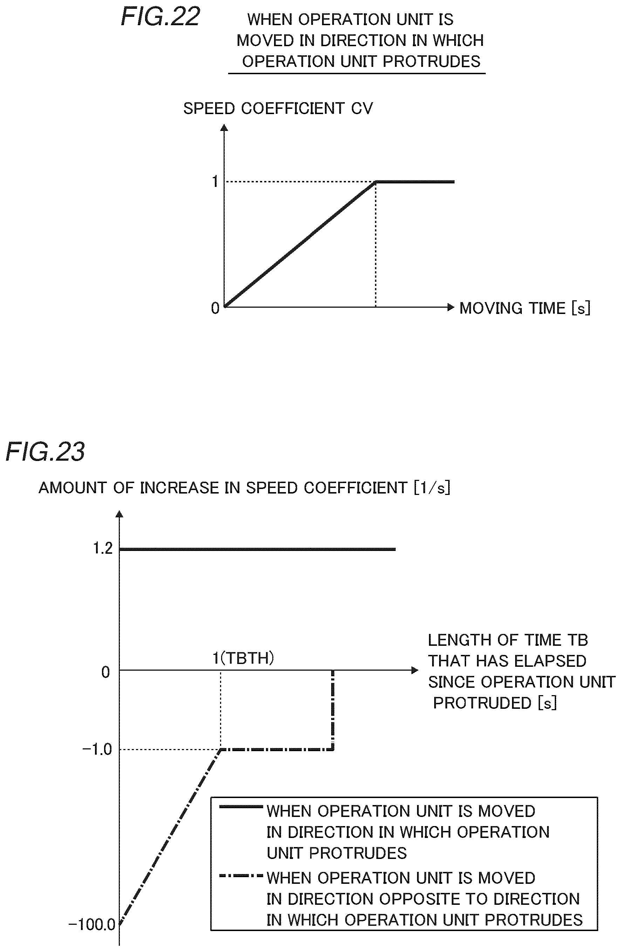

As shown in FIG. 23, the operation controller 340 performs a control to limit the amount of increase or decrease in the speed coefficient CV per unit time. Specifically, when the operation unit 110 is moved in the direction in which the operation unit 110 protrudes from the plane SU into the abnormal region, the operation controller 340 limits the amount of increase in the speed coefficient CV per unit time to a predetermined amount of increase. Therefore, as shown in FIG. 22, when the operation unit 110 is moved in the direction in which the operation unit 110 protrudes from the plane SU into the abnormal region, the speed coefficient CV is increased by the predetermined amount of increase until the speed coefficient CV becomes constant at a maximum value. The predetermined amount of increase is 1.2/s, for example. Furthermore, as shown in FIG. 23, when the operation unit 110 is moved in the direction opposite to the direction in which the operation unit 110 protrudes from the plane SU into the abnormal region, the operation controller 340 performs a control to determine a limited amount of decrease, which is a limit value of the amount of decrease in the speed coefficient CV per unit time, based on the length of time TB that has elapsed since the operation unit 110 protruded, and limit the amount of decrease in the speed coefficient CV per unit time to the determined limited amount of decrease. Specifically, when the length of time TB is greater than zero and equal to or less than a time threshold TBTH, the operation controller 340 reduces the limited amount of decrease as the length of time TB increases. When the length of time TB is greater than the time threshold TBTH, the operation controller 340 maintains the limited amount of decrease constant at a value obtained when the length of time TB is equal to the time threshold TBTH when the speed coefficient of the previous control cycle is less than one, and sets the limited amount of decrease to zero when the speed coefficient of the previous control cycle is one. The time threshold TBTH is 1 s, for example. The operation controller 340 performs a control to determine the speed coefficient CV acquired so as to correspond to the moving speed VE of the operation unit 110 as the speed coefficient CV used to adjust the force PW.

For example, when the length of time TB is greater than zero and equal to or less than the time threshold TBTH, the limited amount of decrease is reduced from - 100/s to -1.0/s. Furthermore, for example, when the length of time TB is greater than the time threshold TBTH and the speed coefficient of the previous control cycle is less than one, the limited amount of decrease is set to - 1.0/s.

When the operation unit 110 is moved in the direction opposite to the direction in which the operation unit 110 protrudes from the plane SU into the abnormal region, the operation controller 340 performs a control to change a calculation method for calculating the speed coefficient CV when the absolute value of the moving speed VE of the operation unit 110 becomes equal to or greater than a predetermined speed threshold that is greater than the speed threshold VTH. That is, when it is determined that the operator is intentionally moving the operation unit 110 in the direction opposite to the direction in which the operation unit 110 protrudes from the plane SU into the abnormal region, the operation controller 340 performs a control to change the calculation method for calculating the speed coefficient CV from a calculation method for calculating the speed coefficient CV so as to correspond to the graph shown in FIG. 21 to the following calculation method. Specifically, the operation controller 340 performs a control to calculate the speed coefficient CV by a formula CV = CV_{c} + (VE - (-VTH_{c}))/100, where CV_{c} represents the speed coefficient CV at the time of change and VTH_{c} represents the predetermined speed threshold. The predetermined speed threshold is 80 mm/s, for example.

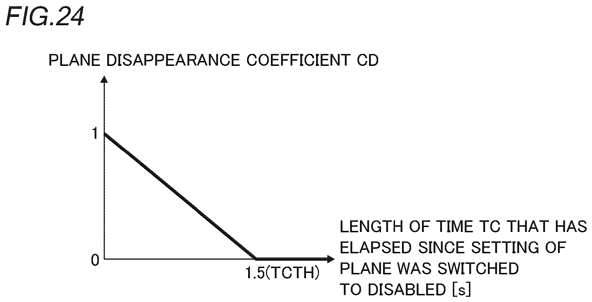

In this embodiment, as shown in FIG. 24, when setting of the plane SU is switched from enabled to disabled, the operation controller 340 gradually reduces the magnitude of the force PW over a predetermined period of time. Specifically, the operation controller 340 performs a control to determine a plane disappearance coefficient CD to adjust the magnitude of the force PW based on the length of time TC that has elapsed since setting of the plane SU was switched from enabled to disabled. Specifically, when the length of time TC is zero, the operation controller 340 sets the magnitude of the plane disappearance coefficient CD to a maximum value, when the length of time TC is greater than zero and is equal to or less than a time threshold TCTH, the operation controller 340 reduces the magnitude of the plane disappearance coefficient CD as the length of time TC increases, and when the length of time TC is greater than the time threshold TCTH, the magnitude of the plane disappearance coefficient CD is set to zero. The plane disappearance coefficient CD has a minimum value of 0 and a maximum value of 1. The time threshold TCTH is 1.5 s, for example. When setting of the plane SU is switched from enabled to disabled due to satisfaction of a predetermined condition, the magnitude of the force PW gradually reduces over a predetermined period of time, as described above. However, when the robot arm 50 to be controlled is switched by operating the switching pedal 122 and the camera pedal 124, for example, setting of the plane SU is switched from enabled to disabled, and the magnitude of the force PW instantly becomes zero.

The operation controller 340 performs a control to acquire the magnitude of the force PW based on the position coefficient CP, the speed coefficient CV, the plane disappearance coefficient CD, and the maximum value of the force PW that is determined in advance. Specifically, the operation controller 340 performs a control to acquire the magnitude of the force PW by a formula PW = CP × CV × CD × PWₘₐₓ, where PWₘₐₓ represents the maximum value of the force PW. Then, the operation controller 340 performs a control to generate a command to apply the force PW of the acquired magnitude, and output the command to the servomotors SM7a, SM7b, and SM7c. Then, when the servomotors SM7a, SM7b, and SM7c are driven based on the command, the force PW is applied as a result of the combination of forces generated by driving of the servomotors SM7a, SM7b, and SM7c. The maximum value of the force PW is 2.5 N, for example.

### Advantages of This Embodiment

The robotic surgical system 500 includes the operation controller 340 configured or programmed to set the plane SU when the operation unit 110 is moved such that the robot arm 50 is attempting to move out of the normal region, and control the servomotors SM7a, SM7b, and SM7c to apply the force PW to the operation unit 110 when the operation unit 110 crosses the plane SU. Accordingly, the force PW acting on the operation unit 110 allows the operator to know that the robot arm 50 is attempting to move out of the normal region, and the force PW acting on the operation unit 110 allows the operator to intuitively know the operation direction of the operation unit 110 to return the robot arm 50 to the normal region. Consequently, the robot arm 50 can be easily returned to the normal region.

The operation controller 340 is configured or programmed to perform a control to determine the position coefficient CP to adjust the magnitude of the force PW based on the protrusion amount AM of the operation unit 110 from the plane SU into the abnormal region. When a relatively large force PW is applied immediately after the operation unit 110 crosses the plane SU, the operation unit 110 is attempted to be moved in a direction in which the operation unit 110 is returned from the plane SU by the relatively large force PW. On the other hand, immediately after the operation unit 110 crosses the plane SU, the operation unit 110 is attempted to be moved by the operator in a direction beyond the plane SU, and thus the operation unit 110 may wobble at the position of the plane SU. Therefore, it is possible to reduce or prevent application of a relatively large force PW immediately after the operation unit 110 crosses the plane SU by adjusting the magnitude of the force PW based on the protrusion amount AM of the operation unit 110 from the plane SU, as described above, and thus wobbling of the operation unit 110 can be reduced or prevented.

The operation controller 340 is configured or programmed to set the magnitude of the position coefficient CP to zero when the protrusion amount AM is zero, and increase the magnitude of the position coefficient CP as the protrusion amount AM increases when the protrusion amount AM is greater than zero. Accordingly, the magnitude of the force PW immediately after the operation unit 110 crosses the plane SU can be relatively reduced, and thus wobbling of the operation unit 110 can be easily reduced or prevented. Furthermore, the magnitude of the force PW can be relatively increased as the protrusion amount AM increases, and thus the possibility that the operation unit 110 moves excessively beyond the plane SU can be reduced or prevented.

The operation controller 340 is configured or programmed to increase the magnitude of the position coefficient CP as the protrusion amount AM increases when the protrusion amount AM is greater than zero and equal to or less than the protrusion amount threshold ATH, and maintains the magnitude of the position coefficient CP constant at a maximum value when the protrusion amount AM is greater than the protrusion amount threshold ATH. Accordingly, when the protrusion amount AM is greater than zero and equal to or less than the protrusion amount threshold ATH, the magnitude of the position coefficient CP is increased as the protrusion amount AM increases such that, as described above, wobbling of the operation unit 110 can be easily reduced or prevented, and the possibility that the operation unit 110 moves excessively beyond the plane SU can be reduced or prevented. Furthermore, when the protrusion amount AM is greater than the protrusion amount threshold ATH, it is possible to reduce or prevent the possibility that an excessively large force PW acts on the operation unit 110 by maintaining the magnitude of the position coefficient CP constant at a maximum value.

The operation controller 340 is configured or programmed to perform a control to determine the speed coefficient CV to adjust the magnitude of the force PW based on the moving speed VE of the operation unit 110. Accordingly, the magnitude of the force PW can be appropriately adjusted based on the moving speed VE of the operation unit 110, and thus wobbling of the operation unit 110 can be reduced or prevented.

The operation controller 340 is configured or programmed to, when the operation unit 110 is moved in the direction opposite to the direction in which the operation unit 110 protrudes from the plane SU into the abnormal region, reduce the magnitude of the speed coefficient CV as the absolute value of the moving speed VE of the operation unit 110 increases when the absolute value of the moving speed VE of the operation unit 110 is greater than zero and equal to or less than the speed threshold VTH, and set the magnitude of the speed coefficient CV to zero when the absolute value of the moving speed VE of the operation unit 110 is greater than the speed threshold VTH. Accordingly, when the operation unit 110 is moved in the direction in which the operation unit 110 is returned from the plane SU by the force PW regardless of the intention of the operator, the magnitude of the force PW of the operation unit 110 can be reduced according to the moving speed VE of the operation unit 110, and thus the possibility that the operation unit 110 is returned excessively can be reduced or prevented. Consequently, wobbling of the operation unit 110 can be reduced or prevented.

The operation controller 340 is configured or programmed to limit the amount of increase in the speed coefficient CV per unit time to the predetermined amount of increase when the operation unit 110 is moved in the direction in which the operation unit 110 protrudes from the plane SU into the abnormal region. Accordingly, the force PW can be gradually increased after the operation unit 110 crosses the plane SU, and thus it is possible to reduce or prevent application of a relatively large force PW immediately after the operation unit 110 crosses the plane SU. Consequently, wobbling of the operation unit 110 can be reduced or prevented.

The operation controller 340 is configured or programmed to perform a control to switch setting of the plane SU between enabled and disabled based on a predetermined condition. Accordingly, if necessary, setting of the plane SU can be enabled to apply the force PW to the operation unit 110, and if not necessary, setting of the plane SU can be disabled not to apply the force PW to the operation unit 110.

The operation controller 340 is configured or programmed to gradually reduce the magnitude of the force PW over the predetermined period of time when setting of the plane SU is switched from enabled to disabled. Accordingly, when the magnitude of the force PW is set to zero immediately after setting of the plane SU is switched from enabled to disabled, the operator may erroneously operate the operation unit 110 due to a sudden loss of the force PW acting on the operation unit 110. However, since the magnitude of the force PW is gradually reduced over the predetermined period of time, the possibility that the operator erroneously operates the operation unit 110 can be reduced or prevented.

The operation controller 340 is configured or programmed to determine the operation direction of the operation unit 110 in which the robot arm 50 returns to the normal region, and control the servomotors SM7a, SM7b, and SM7c to apply the force PW in the determined operation direction. Accordingly, the force PW can be applied in the operation direction of the operation unit 110 in which the robot arm 50 returns to the normal region, and thus the operator can more intuitively know the operation direction of the operation unit 110 for returning the robot arm 50 to the normal region by the force PW applied to the operation unit 110.

The plane SU is flat. Accordingly, the processing load for calculating the plane SU can be reduced as compared with a case in which the plane SU is curved, and thus a process can be quickly and easily performed to set the plane SU.

The plane SU is set based on the boundary points between the normal region and the abnormal region outside the normal region in the coordinate system of the operation unit 110. Accordingly, the plane SU can be set at an appropriate position.

### Modified Examples

The embodiment disclosed this time must be considered as illustrative in all points and not restrictive. The scope of the present disclosure is not shown by the above description of the embodiment but by the scope of claims for patent, and all modifications or modified examples within the meaning and scope equivalent to the scope of claims for patent are further included.

While the plane SU is flat in the aforementioned embodiment, the present disclosure is not limited to this. For example, the plane SU may alternatively be curved.

While a control is performed to cause the servomotors SM7a, SM7b, and SM7c to apply the force PW in the aforementioned embodiment, the present disclosure is not limited to this. For example, as long as it is possible to apply the force PW, a control may alternatively be performed to cause any servomotor to apply the force PW.

While the operation controller 340 performs a control to apply the force PW in the aforementioned embodiment, the present disclosure is not limited to this. In the present disclosure, a component other than the operation controller 340 may alternatively perform a control to apply the force PW. Alternatively, in the aforementioned embodiment, part or all of the control performed by the operation controller 340 may be performed by another controller.

While the magnitude of the position coefficient CP is set to zero when the protrusion amount AM is zero, and the magnitude of the position coefficient CP is increased as the protrusion amount AM increases when the protrusion amount AM is greater than zero in the aforementioned embodiment, the present disclosure is not limited to this. In the present disclosure, the magnitude of the position coefficient CP may alternatively be set to a constant value regardless of the protrusion amount AM.

While when the operation unit 110 is moved in the direction opposite to the direction in which the operation unit 110 protrudes from the plane SU into the abnormal region, the magnitude of the speed coefficient CV is reduced as the absolute value of the moving speed VE of the operation unit 110 increases when the absolute value of the moving speed VE of the operation unit 110 is greater than zero and equal to or less than the speed threshold VTH, and the magnitude of the speed coefficient CV is set to zero when the absolute value of the moving speed VE of the operation unit 110 is greater than the speed threshold VTH in the aforementioned embodiment, the present disclosure is not limited to this. In the present disclosure, when the operation unit 110 is moved in the direction opposite to the direction in which the operation unit 110 protrudes from the plane SU into the abnormal region, the magnitude of the speed coefficient CV may alternatively be constant regardless of the absolute value of the moving speed VE of the operation unit 110. Alternatively, when the operation unit 110 is moved in the direction opposite to the direction in which the operation unit 110 protrudes from the plane SU into the abnormal region, the magnitude of the speed coefficient CV may be reduced as the absolute value of the moving speed VE of the operation unit 110 increases when the absolute value of the moving speed VE of the operation unit 110 is greater than zero and equal to or less than the speed threshold VTH, and the magnitude of the speed coefficient CV may be set to a constant value greater than zero when the absolute value of the moving speed VE of the operation unit 110 is greater than the speed threshold VTH.

While when the operation unit 110 is moved in the direction in which the operation unit 110 protrudes from the plane SU into the abnormal region, the speed coefficient CV is increased by the predetermined amount of increase in the aforementioned embodiment, the present disclosure is not limited to this. In the present disclosure, when the operation unit 110 is moved in the direction in which the operation unit 110 protrudes from the plane SU into the abnormal region, the speed coefficient CV may alternatively be set to a constant value.

While when setting of the plane SU is switched from enabled to disabled, the magnitude of the force PW is gradually reduced over the predetermined period of time in the aforementioned embodiment, the present disclosure is not limited to this. In the present disclosure, when setting of the plane SU is switched from enabled to disabled, the magnitude of the force PW may alternatively be immediately set to zero.

While four robot arms 50 are arranged in the aforementioned embodiment, the present disclosure is not limited to this. In the present disclosure, the number of robot arms 50 may alternatively be other than four.

While each of the arm portion 51 and the positioner 30 includes a 7-axis articulated robot in the aforementioned embodiment, the present disclosure is not limited to this. For example, each of the arm portion 51 and the positioner 30 may alternatively include an articulated robot having an axis configuration other than the 7-axis articulated robot. The axis configuration other than the 7-axis articulated robot refers to six axes or eight axes, for example.

While the surgical robot 100 includes the medical cart 10, the positioner 30, the arm base 40, and the robot arms 50 in the aforementioned embodiment, the present disclosure is not limited to this. For example, the surgical robot 100 may not include the medical cart 10, the positioner 30, or the arm base 40, but may include only the robot arms 50.

The functionality of the elements disclosed herein may be implemented using circuitry or processing circuitry which includes general purpose processors, special purpose processors, integrated circuits, ASICs ("Application Specific Integrated Circuits"), conventional circuitry and/or combinations thereof which are configured or programmed to perform the disclosed functionality. Processors are considered processing circuitry or circuitry as they include transistors and other circuitry therein. The processor may be a programmed processor which executes a program stored in a memory. In the disclosure, the circuitry, units, or means are hardware that carry out or are programmed to perform the recited functionality. The hardware may be any hardware disclosed herein or otherwise known which is programmed or configured to carry out the recited functionality. When the hardware is a processor which may be considered a type of circuitry, the circuitry, means, or units are a combination of hardware and software, the software being used to configure the hardware and/or processor.

### Aspects

It will be appreciated by those skilled in the art that the exemplary embodiments described above are specific examples of the following aspects.

### (Item 1)

A robotic surgical system (500) comprising:
a surgical robot (100) including a robot arm (50) to allow a surgical instrument (1) to be attached thereto;
an operation apparatus (200) including an operation unit (110) to receive an operation from an operator, the operation unit (110) including a drive (SM7a, SM7b, SM7c) to assist the operator in performing the operation, the operation apparatus (200) being configured to operate the surgical robot (100); and
a control device (340) configured or programmed to set a plane (SU) when the operation unit (110) is moved such that the robot arm (50) is attempting to move out of a normal region, and control the drive (SM7a, SM7b, SM7c) to apply a force (PW) to the operation unit (110) when the operation unit (110) crosses the plane (SU).

### (Item 2)

The robotic surgical system (500) according to item 1, wherein the control device (340) is configured or programmed to perform a control to determine a position coefficient (CP) to adjust a magnitude of the force (PW) based on a protrusion amount (AM) of the operation unit (110) from the plane (SU) into an abnormal region.

### (Item 3)

The robotic surgical system (500) according to item 2, wherein the control device (340) is configured or programmed to:
set a magnitude of the position coefficient (CP) to zero when the protrusion amount (AM) is zero; and
increase the magnitude of the position coefficient (CP) as the protrusion amount (AM) increases when the protrusion amount (AM) is greater than zero.

### (Item 4)

The robotic surgical system (500) according to item 3, wherein the control device (340) is configured or programmed to:
increase the magnitude of the position coefficient (CP) as the protrusion amount (AM) increases when the protrusion amount (AM) is greater than zero and equal to or less than a protrusion amount threshold (ATH); and
maintain the magnitude of the position coefficient (CP) constant at a maximum value when the protrusion amount (AM) is greater than the protrusion amount threshold (ATH).

### (Item 5)

The robotic surgical system (500) according to any one of items 1 to 4, wherein the control device (340) is configured or programmed to perform a control to determine a speed coefficient (CV) to adjust a magnitude of the force (PW) based on a moving speed (VE) of the operation unit (110).

### (Item 6)

The robotic surgical system (500) according to item 5, wherein the control device (340) is configured or programmed to, when the operation unit (110) is moved in a direction opposite to a direction in which the operation unit (110) protrudes from the plane (SU) into an abnormal region, reduce a magnitude of the speed coefficient (CV) as an absolute value of the moving speed (VE) of the operation unit (110) increases when the absolute value of the moving speed (VE) of the operation unit (110) is greater than zero and equal to or less than a speed threshold (VTH), and set the magnitude of the speed coefficient (CV) to zero when the absolute value of the moving speed (VE) of the operation unit (110) is greater than the speed threshold (VTH).

### (Item 7)

The robotic surgical system (500) according to item 6, wherein the control device (340) is configured or programmed to limit an amount of increase in the speed coefficient (CV) per unit time to a predetermined amount of increase when the operation unit (110) is moved in the direction in which the operation unit (110) protrudes from the plane (SU) into the abnormal region.

### (Item 8)

The robotic surgical system (500) according to any one of items 1 to 7, wherein the control device (340) is configured or programmed to perform a control to switch setting of the plane (SU) between enabled and disabled based on a predetermined condition.

### (Item 9)

The robotic surgical system (500) according to item 8, wherein the control device (340) is configured or programmed to gradually reduce a magnitude of the force (PW) over a predetermined period of time when the setting of the plane (SU) is switched from enabled to disabled.

### (Item 10)

The robotic surgical system (500) according to any one of items 1 to 9, wherein the control device (340) is configured or programmed to determine an operation direction of the operation unit (110) in which the robot arm (50) returns to the normal region, and control the drive (SM7a, SM7b, SM7c) to apply the force (PW) in a determined operation direction.

### (Item 11)

The robotic surgical system (500) according to any one of items 1 to 10, wherein the plane (SU) is flat.

### (Item 12)

The robotic surgical system (500) according to any one of items 1 to 11, wherein the plane (SU) is set based on boundary points between the normal region and an abnormal region outside the normal region in a coordinate system of the operation unit (110).

### (Item 13)

A control method for a robotic surgical system (500), the robotic surgical system (500) including a surgical robot (100) including a robot arm (50) to allow a surgical instrument (1) to be attached thereto, and an operation apparatus (200) including an operation unit (110) to receive an operation from an operator, the operation unit

(110) including a drive (SM7a, SM7b, SM7c) to assist the operator in performing the operation, the operation apparatus (200) being configured to operate the surgical robot (100), the control method comprising:
receiving an operation on the operation unit (110); and
setting a plane (SU) when the operation unit (110) is moved such that the robot arm (50) is attempting to move out of a normal region and controlling the drive (SM7a, SM7b, SM7c) to apply a force (PW) to the operation unit (110) when the operation unit (110) crosses the plane (SU).

### (Item 14)

A program (341a) configured to cause a computer (340) to execute the control method for the robotic surgical system (500) according to item 13.

### (Item 15)

A storage medium (341) configured to store the program (341a) for the control method for the robotic surgical system (500) according to item 14.

## Claims

1. A robotic surgical system (500) comprising:
a surgical robot (100) including a robot arm (50) to allow a surgical instrument (1) to be attached thereto;
an operation apparatus (200) including an operation unit (110) to receive an operation from an operator, the operation unit (110) including a drive (SM7a, SM7b, SM7c) to assist the operator in performing the operation, the operation apparatus (200) being configured to operate the surgical robot (100); and
a control device (340) configured or programmed to set a plane (SU) when the operation unit (110) is moved such that the robot arm (50) is attempting to move out of a normal region, and control the drive (SM7a, SM7b, SM7c) to apply a force (PW) to the operation unit (110) when the operation unit (110) crosses the plane (SU).

2. The robotic surgical system (500) according to claim 1, wherein the control device (340) is configured or programmed to perform a control to determine a position coefficient (CP) to adjust a magnitude of the force (PW) based on a protrusion amount (AM) of the operation unit (110) from the plane (SU) into an abnormal region.

3. The robotic surgical system (500) according to claim 2, wherein the control device (340) is configured or programmed to:
set a magnitude of the position coefficient (CP) to zero when the protrusion amount (AM) is zero; and
increase the magnitude of the position coefficient (CP) as the protrusion amount (AM) increases when the protrusion amount (AM) is greater than zero.

4. The robotic surgical system (500) according to claim 3, wherein the control device (340) is configured or programmed to:
increase the magnitude of the position coefficient (CP) as the protrusion amount (AM) increases when the protrusion amount (AM) is greater than zero and equal to or less than a protrusion amount threshold (ATH); and
maintain the magnitude of the position coefficient (CP) constant at a maximum value when the protrusion amount (AM) is greater than the protrusion amount threshold (ATH).

5. The robotic surgical system (500) according to any one of claims 1 to 4, wherein the control device (340) is configured or programmed to perform a control to determine a speed coefficient (CV) to adjust a magnitude of the force (PW) based on a moving speed (VE) of the operation unit (110).

6. The robotic surgical system (500) according to claim 5, wherein the control device (340) is configured or programmed to, when the operation unit (110) is moved in a direction opposite to a direction in which the operation unit (110) protrudes from the plane (SU) into an abnormal region, reduce a magnitude of the speed coefficient (CV) as an absolute value of the moving speed (VE) of the operation unit (110) increases when the absolute value of the moving speed (VE) of the operation unit (110) is greater than zero and equal to or less than a speed threshold (VTH), and set the magnitude of the speed coefficient (CV) to zero when the absolute value of the moving speed (VE) of the operation unit (110) is greater than the speed threshold (VTH).

7. The robotic surgical system (500) according to claim 6, wherein the control device (340) is configured or programmed to limit an amount of increase in the speed coefficient (CV) per unit time to a predetermined amount of increase when the operation unit (110) is moved in the direction in which the operation unit (110) protrudes from the plane (SU) into the abnormal region.

8. The robotic surgical system (500) according to any one of claims 1 to 7, wherein the control device (340) is configured or programmed to perform a control to switch setting of the plane (SU) between enabled and disabled based on a predetermined condition.

9. The robotic surgical system (500) according to claim 8, wherein the control device (340) is configured or programmed to gradually reduce a magnitude of the force (PW) over a predetermined period of time when the setting of the plane (SU) is switched from enabled to disabled.

10. The robotic surgical system (500) according to any one of claims 1 to 9, wherein the control device (340) is configured or programmed to determine an operation direction of the operation unit (110) in which the robot arm (50) returns to the normal region, and control the drive (SM7a, SM7b, SM7c) to apply the force (PW) in a determined operation direction.

11. The robotic surgical system (500) according to any one of claims 1 to 10, wherein the plane (SU) is flat.

12. The robotic surgical system (500) according to any one of claims 1 to 11, wherein the plane (SU) is set based on boundary points between the normal region and an abnormal region outside the normal region in a coordinate system of the operation unit (110).

13. A control method for a robotic surgical system (500), the robotic surgical system (500) including a surgical robot (100) including a robot arm (50) to allow a surgical instrument (1) to be attached thereto, and an operation apparatus (200) including an operation unit (110) to receive an operation from an operator, the operation unit (110) including a drive (SM7a, SM7b, SM7c) to assist the operator in performing the operation, the operation apparatus (200) being configured to operate the surgical robot (100), the control method comprising:
receiving an operation on the operation unit (110); and
setting a plane (SU) when the operation unit (110) is moved such that the robot arm (50) is attempting to move out of a normal region and controlling the drive (SM7a, SM7b, SM7c) to apply a force (PW) to the operation unit (110) when the operation unit (110) crosses the plane (SU).

14. A program (341a) configured to cause a computer (340) to execute the control method for the robotic surgical system (500) according to claim 13.

15. A storage medium (341) configured to store the program (341a) for the control method for the robotic surgical system (500) according to claim 14.
